# EUROPEAN PATENT SPECIFICATION

(11) **EP 1 637 076 B1**
(45) Date of publication and mention of the grant of the patent: **18.03.2015**
(21) Application number: 05255589.3
(22) Date of filing: 13.09.2005
(51) Int. Cl.: A61B 5/0492, A61B 5/04

(54) **Sensor system for detecting and processing EMG signals**
Sensorsystem zur Ermittlung und Verarbeitung elektromygrafischer Signale
Système de capteur pour détecter et traiter les signaux électromygraphiques

(30) Priority: 16.09.2004 US 610435 P
(43) Date of publication of application: 22.03.2006
(73) Proprietor: Altec, Inc., Boston, MA 02215 (US)
(72) Inventor: DeLuca, Carlo J., Wellesley, Massachusetts 02181 (US); Gilmore, Donald L., Wellesley, Massachusetts 02181 (US)
(74) Representative: Freischem, Stephan

(56) References cited:
- WO-A-2004/023996
- US-A- 6 004 312
- US-A1- 2002 147 411
- US-A1- 2003 069 514
- TZYH-YI SUN ET AL: "Clinical applications of surface EMG multielectrode for diagnosis of neuromuscular diseases" CHINESE JOURNAL OF MEDICAL AND BIOLOGICAL ENGINEERING TAIWAN, vol. 14, no. 3, September 1994 (1994-09), pages 211-222, XP008055844 ISSN: 1019-0465
- BLOK J H ET AL: "A high-density multichannel surface electromyography system for the characterization of single motor units" REVIEW OF SCIENTIFIC INSTRUMENTS, AMERICAN INSTITUTE OF PHYSICS, US, vol. 73, no. 4, April 2002 (2002-04), pages 1887-1897, XP012039983 ISSN: 0034-6748
- DE LA BARRARA E J ET AL: "A Sensitive Technique To Optimize Recording Of Surface EMG Signal" ENGINEERING IN MEDICINE AND BIOLOGY SOCIETY, 1991. VOL.13: 1991., PROCEEDINGS OF THE ANNUAL INTERNATIONAL CONFERENCE OF THE IEEE ORLANDO, FL, USA 31 OCT.-3 NOV. 1991, NEW YORK, NY, USA,IEEE, US, 31 October 1991 (1991-10-31), pages 837-838, XP010101728 ISBN: 0-7803-0216-8
- POZZO M ET AL: "SIXTY-FOUR CHANNEL WEARABLE ACQUISITION SYSTEM FOR LONG-TERM SURFACE ELECTROMYOGRAM RECORDING WITH ELECTRODE ARRAYS" MEDICAL AND BIOLOGICAL ENGINEERING AND COMPUTING, PETER PEREGRINUS, STEVENAGE, GB, vol. 42, no. 4, July 2004 (2004-07), pages 455-466, XP001221029 ISSN: 0140-0118

## Description

### BACKGROUND OF THE INVENTION

Medical discipline employs armament for diagnostics, quantitative objective techniques and tests to evaluate degrees of insult or dysfunction. The object of this invention is to utilize such techniques in the field of motor disorders. Each year approximately one million Americans are struck with a debilitating motor disorder or are afflicted with a disease which impairs their ability to move, carry out normal activities of daily living, and in various ways degrade their quality of life. The most common disorders among these are Stroke, Spinal Cord Injuries, Head Injuries, Parkinson's Disease, Multiple Sclerosis, and various forms of paralysis, such as facial palsy. Although neural lesions associated with upper motoneuron disorders can be imaged with MRI and fMRI magnets to indicate the location and size of the lesion, the images do not provide a diagnostic assessment of the degree of impairment and the degree of recovery.

In addition to these "upper motoneuron" disorders, there are countless "lower motoneuron" dysfunctions such as myestinea gravis. Peripheral nerve injuries caused by trauma and accident, and an annually increasing number of neuromuscular dysfunctions due to neurotoxins in our environment such as Organophosphate based pesticides and insecticides. These later dysfunctions are typically assessed with procedures that require repeated insertion of needles into muscles and probe the tissues for signs of abnormal action potentials. Although numerous attempts have been made to quantify the parameter of the action potentials the procedure remains essentially subjective and very much dependent on the skill and perseverance of the clinician because the procedure is painful and only one or two action potentials are commonly obtained at each site that is tested. The techniques used for these tests have remained essentially unchanged for the past four decades. Patients find these tests stressful and the collected data is often inconclusive because of the limited size and often poor quality.

The EMG signal is composed of the action potentials (or electrical pulses) from groups of muscle fibers (grouped into functional units called motor units). Refer to the book Muscles Alive (5 Th.Ed, 1985) for details. The signal is detected with electrodes placed on the surface of the skin or with needle or wire electrodes introduced into the muscle tissue. The term decomposition is commonly used to describe the process whereby individual motor unit action potentials (MUAPs) are identified and uniquely classified from a set of superimposed motor unit action potentials which constitute the EMG signal. A decomposed EMG signal provides all the information available in the EMG signal. The timing information provides a complete description of the inter-pulse interval, firing rate and synchronization characteristics. The morphology of the shapes of the MUAPs provides information concerning the anatomy and health of the muscle fibers.

To date, all techniques that have been able to identify individual action potentials in the superimposed EMG signal and provide useful physiological information have used indwelling electrodes to detect the signal.

Most recently, a quadrifilar indwelling EMG electrode has been used to collect three channels of EMG signals that could be decomposed, partially automatically, to reveal novel aspects of the behavior of the motor unit control properties. The needle version has the advantage of being repositioned after an insertion or being relocated, thereby increasing the probability of obtaining a quality signal that can be decomposed.

Recently introduced was a wire-electrode version of the quadrifilar electrode. The wire version possesses two advantages: 1) it may be placed in deep muscles located under an overlying layer of muscle, and 2) it generally provides no sensation of discomfort once inserted. But, it has some disadvantages. Once inserted it cannot be precisely relocated within the muscle. One can pull the wire out fractions of a millimeter, but this procedure can only be done once or twice and with little control over the precise placement of the electrode. Both of these types of electrodes have the inherent limitations that:
1. They must be inserted into the muscle. This requires a clinical preparation involving sterilization of the electrodes and the needles, sterilization of the environment where the insertion is to be made.
2. They carry the, albeit low, risk of infection.
3. They cause minor damage to the muscle tissue from which they are detecting the signal.
4. They are not well tolerated by individuals who have needle aversion, such as children.
5. Once these electrodes are inserted, the subject must remain very steady. A minor movement of 0.1 mm may cause the shapes of the motor unit action potentials to change, thus precluding the continued identification of a specific unit and generally incapacitating the decomposition algorithms from identifying actions potentials in the remainder of the contraction.

In addition to these technical limitations, some muscles have not been subjected to investigation because needle insertions would be too dangerous or impractical. For example, the motor unit firing properties of muscles of the lips, eye lids, tongue and most facial muscles have never been investigated.

In US Patent 6,004,312, a generally flat, rectangular rubber pad supports (63) discrete, non-invasive electrodes in a 7 by 9 array and a ground electrode to collect surface electromyographic (EMG) signals from an underlying muscle group of a patient. Electronic apparatus conditions the EMG signals and is programmed to survey the electrode array simultaneously to provide a description of the activity of individual muscles in the muscle group at that point in time. The description is displayed on a video monitor or the like, juxtaposed over a display of normal muscle anatomy, with differences being emphasized by color enhancement. The electrode pad is particularly suited for the lower back muscle groups and may be retained in position by a lumbar support belt having a pad molded to the contours of the human spine.

E. J. de la Barrara et al describe in "A SENSITIVE TECHNIQUE TO OPTIMIZE RECORDING OF SURFACE EMG SIGNAL" a technique that allows the user to quickly find muscle fiber direction and to position electrodes at points where maximal surface EMG (SEMG) activity can be obtained while minimizing unwanted signals. SEMG signals were recorded simultaneously from arrays of colinear bipolar electrodes. Pairs of colinear contacts were spaced 15 mm apart and oriented along lines 26 degrees apart.

The object of this invention, therefore, encompasses a surface array electrode sensor that can detect Electromyographic (EMG) signals consisting of identifiable individual action potentials, the characteristics of which are useful for clinical diagnosis. Additionally, when the electrode array is used in conjunction with special technology and signal processing algorithms it will provide an accurate account of the firing times of each action potential belonging to a motor unit. This information will describe the state of the muscle and the Central Nervous System in a manner that is superior to that currently available by techniques in common practice. Although an important application of the surface sensor would be for clinical use, it has applications in other areas such as: 1) Space Medicine - - where it is of interest to understand if the control of muscles is altered during and after prolonged exposures to microgravity, 2) Ergonomics - - where it is important to learn how muscles are controlled during sustained and/or repetitive tasks so that they may be protected from damage, and 3) Aging - - where it is useful to understand how the control to muscle fibers is altered during the process of aging so that techniques and pharmaceuticals could be developed to counteract the process of aging, and, 4) Physiology - - where it will provide a new tool for understanding how muscles are controlled.

### SUMMARY OF THE INVENTION

The object of this invention is attained by the features of independent claim 1. The dependent claims propose useful embodiments of the invention.

The invention is a sensor system for detecting and processing EMG signals including a substrate having a bottom surface adapted for attachment to skin; a plurality of spaced apart electrode arrays projecting from the bottom surface so as to engage the skin and detect EMG signals in muscles located under the substrate; and four differential amplifiers connected to receive EMG signals from four distinct pairs of electrode arrays. The electrode arrays detect the action potentials of the muscle fibers from various orientations so that the shape of an action potential appears substantially dissimilar in each of the four differential pairs. Because of the greater dissimilarity of the shapes of the same action potential, that the compound electrical signal detected from the arrays can be decomposed into individual action potentials.

According to one feature of the invention, the distinct pairs of electrode arrays are spaced apart in different directions on the substrate. This feature provides particularly valuable test data.

According to another feature, the distinct pairs of electrode arrays are arranged in an orthogonal pattern. This arrangement provides two orthogonal perspectives of the action potential emanating from fibers that traverse the interior of the array perimeter. The different media in these two directions provides substantially different filtering effects on the action potential, resulting in desirable wave shapes that have different spectral and time dependent characteristics.

According to yet another feature, the substrate is elongated in one of the orthogonal directions of said pattern. This feature assists in properly aligning the substrate over muscle being tested.

According to a further feature, the distinct pairs of electrode arrays are arranged in a radial pattern. This arrangement provides two 45 degrees shifted orthogonal perspectives of the action potential emanating from fibers that traverse the interior of the array perimeter to accommodate the orientation of muscle fibers that are not orthogonal to the perimeter of the array.

According to another feature, the distinct pairs of electrode arrays include two pairs spaced apart in first aligned directions and two pairs spaced apart in second aligned directions substantially parallel to the first directions. This array arrangement is sensitive to the varying electrical properties of the tissues surrounding the muscle fibers along their length which will have different filtering effects on the action potential.

According to other important features of the invention, the electrode arrays comprise pins with rounded tips, a uniform diameter in the range between .3mm and 1 mm and a projection length of approximately 2mm, the system includes decomposition circuitry connected to receive the four channel signal output from the amplifiers; and the substrate is flexible to accommodate flexing over the skin. These features assist further in providing valuable test data.

### DESCRIPTION OF THE DRAWINGS

These and other objects and features of the invention will become more apparent upon a perusal of the following description taken in conjunction with the accompanying drawings wherein:
Fig. 1 is a block diagram illustrating one embodiment of the invention;
Fig. 2 is a schematic top view of an electrode array used in the embodiment of Fig. 1;
Fig. 3 is a side view of the electrode array shown in Fig. 2;
Fig. 4 is a block diagram of another embodiment of the invention;
Fig. 5 is a schematic top view of an electrode array used in the embodiment of Fig. 4;
Fig. 6 is a side view of the electrode array shown in Fig. 5;
Fig. 7 is a block diagram of an embodiment in which two pairs of electrodes are spaced apart in first aligned directions and two pairs of electrodes are spaced apart in second aligned directions parallel to the first directions;
Fig. 8 is a schematic top view of an electrode array used in the embodiment of Fig. 7;
Fig. 9 is a side view of the electrode array shown in Fig. 8; and
Fig. 10 illustrates four channels of differential signal pairs provided by the embodiment of Figs. 1-3.

### DESCRIPTION OF THE PREFERRED EMBODIMENTS

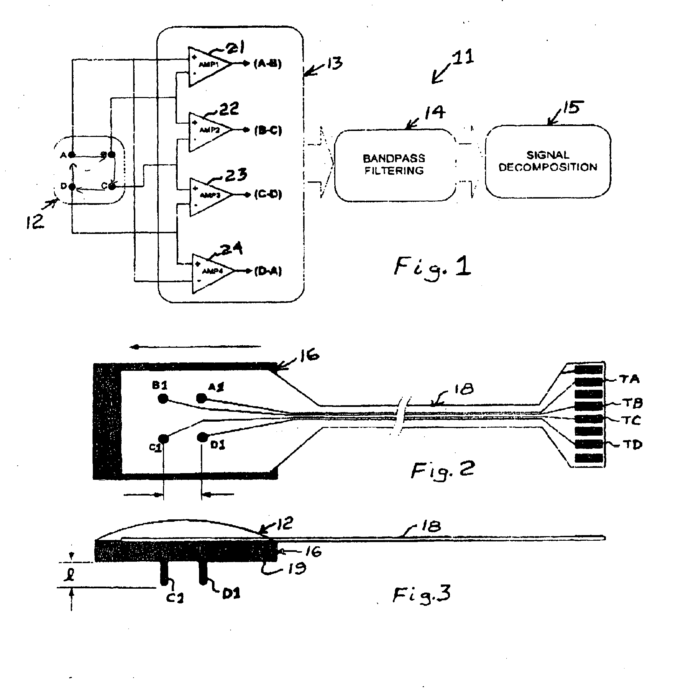

A system 11 for detecting and processing EMG signals is shown in the block diagram of Fig. 1 and includes an electrode section 12, an amplifier section 13, a filtering section 14 and a decomposing section 15. Included in the section 12 is an electrode array (Fig. 2) composed of electrodes A1, B1, C1, and D1 uniformly spaced apart on a rectangular substrate 16. Connected by a cable 18 to the electrodes A1, B1, E1 and D1 are, respectively, output terminals TA, TB, TC and TD. As shown in Fig. 3, the electrodes are pins having rounded ends and projecting a length ℓ of 2mm from a bottom surface 19 of the substrate 16. The terminals TA - TD are connected to section 13 with terminals TA and TB connected to a differential amplifier 21, terminals TB and TC connected to a differential amplifier 22, terminals TC and TD connected to a differential amplifier 23, and terminals TD and TA connected to a differential amplifier 24. Preferably, the pin electrodes A1 - D1 are uniformly spaced apart, as shown, in an orthogonal array with a uniform spacing of between 1.5mm and 5mm, preferably a distance of about 3.6mm. Also, all of the pin electrodes have a diameter of between .3mm and 1 mm.

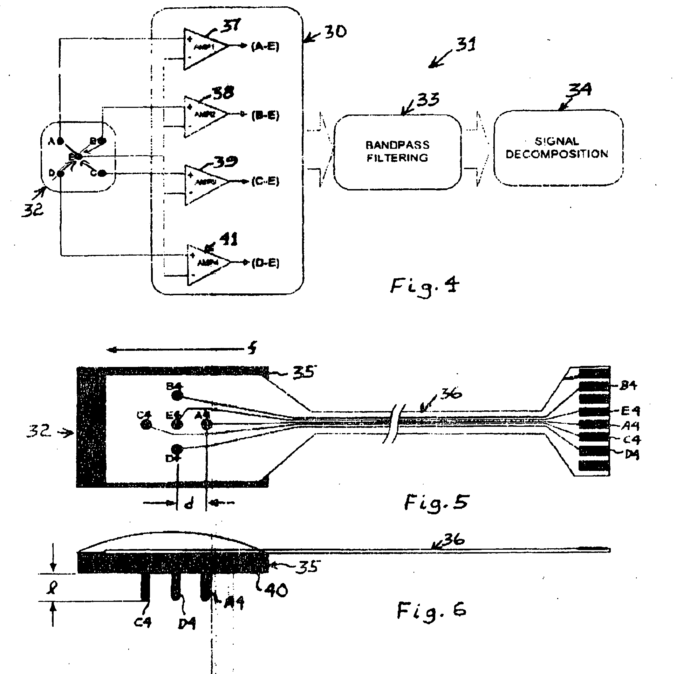

Fig. 4 depicts another sensor system 31 in which another electrode section embodiment 32 is connected to an amplifier section 30, a filtering section 33 and a decomposing section 34. Included in the section 32 is an electrode array (Fig. 5) composed of electrodes A4, B4, C4, D4 and E4 spaced apart on a rectangular substrate 35. Connected by a cable 36 to the electrodes A4 - E4 are, respectively, output terminals TA, TB, TC, TD and TE. As shown in Fig. 6, the electrodes are pins having rounded ends and projecting a length ℓ of 2mm from a bottom surface 35 of the substrate 33. The terminals TA - TE are connected to the amplifier section 30 a section 13 with terminals TA and TE connected to a differential amplifier 37, terminals TB and TE connected to a differential amplifier 38, terminals TC and TE connected to a differential amplifier 39 and terminals TD and TE connected to a differential amplifier 41. Preferably, the electrode pins A4 - D4 are uniformly spaced from the electrode pin E4 in a radial array and with a uniform spacing of between 1.5mm and 5mm and preferably a distance d of about 3.6 mm. Also, all of the pins have a diameter of between .3mm and 1mm.

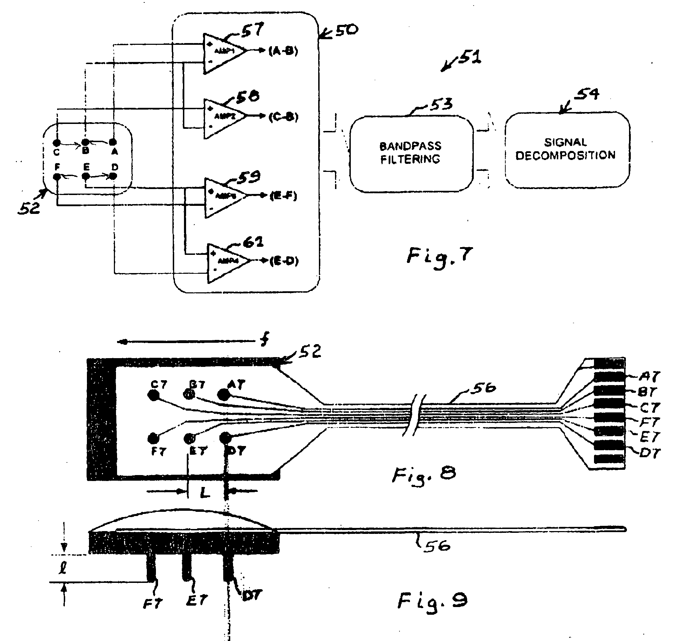

Fig. 7 illustrates another sensor system 51 in which an electrode section embodiment 52 is connected to an amplifier section 50, a filtering section 53, and a decomposing section 54. Included in the section 52 is an electrode array (Fig. 8) composed of electrodes A7, B7, C7, D7, E7 and F7 spaced apart on a rectangular substrate 55. Connected by a cable 56 to the electrodes A4 - F7 are, respectively, output terminals TA, TB, TC, TD, TE and TF. As shown in Fig. 6, the electrodes are pins having rounded ends and projecting a length ℓ of 2mm from a bottom surface 60 of the substrate 33. The terminals TA - TF are connected to amplifier section 50 with terminals TA and TB connected to a differential amplifier 57, terminals TB and TC connected to a differential amplifier 58, terminals TF and TE connected to a differential amplifier 59 and terminals TD and TE connected to a differential amplifier 61. Preferably, the electrode pin pairs A7 and B7, B7 and C7, E7 and F7, and D7 and E7 are uniformly spaced apart with a spacing of between 1.5mm and 5mm and preferably by a distance L of about 2.54mm. Also, all of the pins again have a diameter of between .3mm and 1 mm.

In use, one of the surface array electrodes 12, 32 or 52 is placed on the skin above the muscle of interest. The electrode selected is determined by both the muscle characteristics to be tested and the particular muscle under test. For example, the electrode array 32 of Figs. 4 - 6 is especially effective when used over muscles with non-parallel fibers such as panate muscle, or the electrode array 52 is especially effective when tests of movement of action potentials along parallel muscle fibers f (Figs. 4 and 7) are being made. Sufficient pressure is provided to establish good electrical contact as evidenced by the best signal-to-noise ratio of the detected signals. Good electrical contact is accomplished by viewing the detected signal on a computer screen in real time. However, if the signal to noise ratio is poor, it can be improved by applying conductive gel to the tip of the pins. In a typical test, for example, the leads from the electrode pins A1 - D1 are connected to the inputs of the differential amplifiers 21 - 24. A subject or patient is then asked to contract a muscle of interest and over which the substrate 13 is placed. The signals from the surface electrode array 12 are then stored. Next the signals are conditioned by bandpass filtering, usually from 250 Hz to 2kHz in the section 14 in order to remove any movement artifact at the low end of the spectrum and any excessively long tail that some action potentials have. Depending on the configuration of the electrode array that is used and on the particular muscle being tested, the bandwidth may vary from 100 to 2,000 Hz.

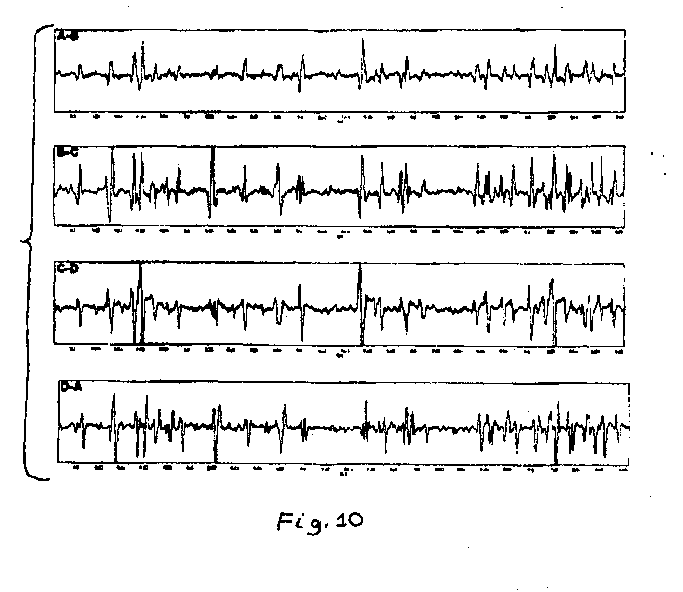

Fig. 10 illustrates four channels of differential EMG signals detected by the electrode array 12 of Fig. 1. The four channels are differential signals provided by the amplifiers 21 - 24 from the electrode pairs A1 - B1, B1 - C1, C1 - D2, and D1 - A2. The signals were detected from the First Dorsal Interosseous muscle in the hand of a male subject. Note that the individual action potentials (pulses) derived from the muscle are clearly visible and identifiable. Some superposition of action potentials from different motor units (having different shapes) does occur. These superpositions, as well as other alterations in the signal, such as gradual modifications in the shape of the action potentials from a particular train, similarities in the shapes of motor units from different motor units, etc. are resolved via special decomposition algorithms.

Obviously, many modifications and variations of the present invention are possible in light of the above teachings. It is to be understood, therefore, that the invention can be practiced otherwise than as specifically described.

## Claims

1. A sensor system for detecting and processing EMG signals comprising:
a substrate (16, 35) having a bottom surface (19, 40) adapted for attachment to skin;
a plurality of spaced apart electrodes (A1 - D1; A4 - E4) projecting from said bottom surface so as to engage the skin and detect EMG signals in a muscle located under said substrate, said electrodes (A1 - D1; A4 - E4) being arranged to provide four distinct pairs (A1,B1; B1,C1; C1,D1; D1,A1 or A4,E4; B4,E4; C4,E4; D4,E4) of said electrodes, wherein said distinct pairs of said electrodes (A1 - D1; A4 - E4) are spaced apart in different directions on said substrate;
an amplifier section (13,30), a filtering section (14,33) and a decomposing section (15,34) connected to said electrodes (A1 - D1; A4 - E4),
the amplifier section (13,30) comprising four differential amplifiers (21 - 24, 37,38,39,41;) each connected to receive EMG signals from one of the distinct pairs of said electrodes (A1 - D1; A4 - E4);
the decomposing section (15,34) comprising decomposition circuitry connected to receive the four channel signal output from the differential amplifiers (21 - 24, 37,38,39,41),
wherein each of the distinct pairs (A1,B1; B1,C1; C1,D1; D1,A1 or A4,E4;
B4,E4; C4,E4; D4,E4) of said electrodes (A1 - D1; A4 - E4) is configured to detect the action potentials of the muscle fibers from a different orientation so that the shape of an action potential appears substantially dissimilar in each of the four distinct pairs; and
wherein the decomposition circuitry (15,34) is configured to decompose the compound electrical signal detected from the electrodes' output signals into individual action potentials.

2. A sensor system according to claim 1 wherein said distinct pairs of electrodes (21-24) are arranged in an orthogonal pattern.

3. A sensor system according to claim 2 wherein said substrate is elongated in one of the orthogonal directions of said pattern.

4. A sensor system according to claim 1, 2 or 3, wherein said distinct pairs of electrodes (A4 - E4) are arranged in a radial pattern with a first electrode (E4) and four electrodes (A4 - D4) being uniformly spaced from said first electrode (E4) in a radial array.

5. A sensor system according to claim 4, wherein each of said amplifiers is connected to said first electrode (E4) and one of said four electrodes (A4, B4, C4, D4) being uniformly spaced from said first electrode (E4) in a radial array.

6. A sensor system according to one of the preceding claims wherein said electrodes (21 - 24; A4 - E4) are uniformly spaced apart a distance in the range between 1.5mm and 5mm.

## Patentansprüche

1. Sensorensystem zur Erfassung und Verarbeitung von EMG-Signalen, umfassend:
ein Substrat (16, 35) mit einer unteren Fläche (19, 40), welche zur Befestigung an der Haut ausgelegt ist;
eine Mehrzahl voneinander beabstandeter Elektroden (A1 - D1 ; A4 - E4), welche aus der genannten unteren Fläche herausragen, um in die Haut einzugreifen und EMG-Signale in einem unter dem genannten Substrat befindlichen Muskel zu erfassen, wobei die genannten Elektroden (A1 - D1 ; A4 - E4) so angeordnet sind, dass sie vier einzelne Paare (A1,B1 ; B1,C1 ; C1,D1; D1,A1 oder A4,E4; B4,E4; C4,E4; D4,E4) der genannten Elektroden ergeben, wobei die genannten einzelnen Paare der genannten Elektroden (A1 - D1 ; A4 - E4) auf dem genannten Substrat in unterschiedlichen Richtungen voneinander beabstandet sind;
einen Verstärker-Abschnitt (13, 30), einen Filter-Abschnitt (14, 33) und einen Zerlegungs-Abschnitt (15, 34), welche mit den genannten Elektroden (A1 - D1 ; A4 - E4) verbunden sind;
wobei der Verstärker-Abschnitt (13, 30) vier Differentialverstärker (21 - 24, 37, 38, 39, 41) umfasst, von denen jeder verbunden ist, um EMG-Signale von einem der einzelnen Paare der genannten Elektroden (A1 - D1 ; A4 - E4) zu empfangen;
wobei der Zerlegungs-Abschnitt (15, 34) eine Zerlegungsschaltung umfasst, welche verbunden ist, um das von den Differentialverstärkern (21 - 24, 37, 38, 39, 41) ausgegebene Vierkanal-Signal zu empfangen,
wobei jedes der einzelnen Paare (A1 ,B1; B1,C1 ; C1,D1 ; D1,A1 oder A4,E4; B4,E4; C4,E4; D4,E4) der genannten Elektroden (A1 - D1 ; A4 - E4) so beschaffen ist, dass es die Aktionspotentiale der Muskelfasern einer unterschiedlichen Orientierung erfasst, so dass die Form eines Aktionspotentials bei jedem der vier einzelnen Paare im Wesentlichen unähnlich erscheint;
wobei die Zerlegungsschaltung (15, 34) so beschaffen ist, dass sie das von den Ausgabesignalen der Elektroden erfasste zusammengesetzte Signal in einzelne Aktionspotentiale zerlegt.

2. Sensorensystem nach Anspruch 1, wobei die genannten einzelnen Paare von Elektroden (21 - 24) in einem orthogonalen Muster angeordnet sind.

3. Sensorensystem nach Anspruch 2, wobei das genannte Substrat in einer der orthogonalen Richtungen des genannten Musters verlängert ist.

4. Sensorensystem nach Anspruch 1, 2 oder 3, wobei die genannten einzelnen Paare von Elektroden (A4 - E4) in einem radialen Muster mit einer ersten Elektrode (E4) und vier Elektroden (A4 - D4), welche gleichmäßig von der genannten ersten Elektrode (E4) in einer radialen Anordnung beabstandet sind, angeordnet sind.

5. Sensorensystem nach Anspruch 4, wobei jeder der genannten Verstärker mit der genannten ersten Elektrode (E4) und einer der genannten vier Elektroden (A4, B4, C4, D4) verbunden ist, welche gleichmäßig von der genannten ersten Elektrode (E4) in einer radialen Anordnung beabstandet sind.

6. Sensorensystem nach einem der vorangehenden Ansprüche, wobei die genannten Elektroden (21 - 24; A4 - E4) um eine Entfernung im Bereich zwischen 1,5 mm und 5 mm gleichmäßig voneinander beabstandet sind.

## Revendications

1. Système de capteur pour détecter et traiter des signaux électro-myographiques (EMG), comprenant :
un substrat (16, 35) présentant une surface inférieure (19, 40) adaptée pour être attachée à la peau ;
une pluralité d'électrodes espacées les unes des autres (A1 - D1 ; A4 - E4) qui font saillie à partir de ladite surface inférieure de manière à engager la peau et à détecter des signaux EMG dans un muscle qui se trouve en dessous dudit substrat, lesdites électrodes (A1 - D1 ; A4 - E4) étant disposées de manière à former quatre paires distinctes (A1,B1; B1,C1; C1,D1; D1,A1 ou A4,E4; B4,E4; C4,E4; D4,E4) desdites électrodes, dans lequel lesdites paires distinctes desdites électrodes (A1 - D1 ; A4 - E4) sont espacées les unes des autres dans des directions différentes sur ledit substrat ;
une section d'amplificateur (13, 30), une section de filtrage (14, 33) et une section de décomposition (15, 34) qui sont connectées auxdites électrodes (A1 - D1 ; A4 - E4) ;
la section d'amplificateur (13, 30) comprenant quatre amplificateurs différentiels (21 - 24, 37, 38, 39, 41) connectés chacun pour recevoir des signaux EMG en provenance de l'une des paires distinctes desdites électrodes (A1 - D1; A4 - E4) ;
la section de décomposition (15, 34) comprenant un circuit de décomposition qui est connecté pour recevoir les quatre signaux de sortie de canal en provenance des amplificateurs différentiels (21 - 24, 37, 38, 39, 41),
dans lequel chacune des paires distinctes (A1,B1 ; B1,C1 ; C1,D1 ;
D1,A1 ou A4,E4 ; B4,E4 ; C4,E4 ; D4,E4) desdites électrodes (A1 - D1 ; A4 - E4) est configurée pour détecter les potentiels d'action des fibres musculaires à partir d'une orientation différente, de telle sorte que la forme d'un potentiel d'action apparaisse sensiblement dissemblable dans chacune des quatre paires distinctes ; et
dans lequel le circuit de décomposition (15, 34) est configuré de manière à décomposer le signal électrique composé détecté à partir des signaux de sortie des électrodes en potentiels d'action individuels.

2. Système de capteur selon la revendication 1, dans lequel lesdites paires distinctes d'électrodes (21 - 24) sont disposées en un motif orthogonal.

3. Système de capteur selon la revendication 2, dans lequel ledit substrat est allongé dans l'une des directions orthogonales dudit motif.

4. Système de capteur selon la revendication 1, 2 ou 3, dans lequel lesdites paires distinctes d'électrodes (A4 - E4) sont disposées en un motif radial avec une première électrode (E4) et quatre électrodes (A4 - D4) qui sont espacées uniformément de ladite première électrode (E4) en un réseau radial.

5. Système de capteur selon la revendication 4, dans lequel chacun desdits amplificateurs est connecté à ladite première électrode (E4) et à l'une desdites quatre électrodes (A4, B4, C4, D4) qui sont espacées uniformément de ladite première électrode (E4) en un réseau radial.

6. Système de capteur selon l'une quelconque des revendications précédentes, dans lequel lesdites électrodes (21 - 24 ; A4 - E4) sont espacées uniformément les unes des autres d'une distance qui est comprise dans la gamme de 1,5 mm à 5 mm.
